# EUROPÄISCHE PATENTANMELDUNG

(11) **EP 3 958 274 A1**
(43) Veröffentlichungstag der Anmeldung: **23.02.2022**
(21) Anmeldenummer: 21000225.9
(22) Anmeldetag: 10.08.2021
(51) Int. Cl.: G16H 20/40, G16H 40/60

(54) **VERFAHREN ZUR UNTERSTÜTZUNG DES BENUTZERS EINES BEATMUNGSGERÄTS UND BEATMUNGSSYSTEM**

(30) Priorität: 18.08.2020 DE 102020121680
(71) Anmelder: Löwenstein Medical Technology S.A., 2557 Luxembourg (LU)
(72) Erfinder: Schwaibold, Matthias, 76228 Karlsruhe (DE)
(74) Vertreter: Marx, Thomas

(57) **Zusammenfassung**

Verfahren zur Unterstützung eines Benutzenden eines Beatmungsgeräts (1), umfassend die nachfolgenden Schritte.
- Koppeln eines einen Steueralgorithmus umfassenden mobilen Endgeräts (2) mit dem Beatmungsgerät (1) zum Austausch von Daten zwischen dem Endgerät (2) und dem Beatmungsgerät (1);
- Übertragen von auf dem Beatmungsgerät (1) registrierten Daten an das Endgerät (2), wobei die Daten einen durch das Beatmungsgerät (1) erfassten Beatmungsparameter umfassen;
- Auswerten der übermittelten Daten auf dem Endgerät (2) und/oder mittels einer mit dem Endgerät (2) kommunizierenden Rechnereinrichtung zur Bereitstellung wenigstens einer Benutzerinformation, sodass der Benutzende unabhängig von seinem medizinischen Fachwissen eine wenigstens teilweise auf dem Beatmungsparameter basierende Bewertung und/oder Hilfestellung erhält;
- Ausgeben der Benutzerinformation mittels des Endgeräts (2).

## Beschreibung

Die vorliegende Erfindung betrifft ein Verfahren zur Unterstützung eines Benutzenden eines Beatmungsgeräts und insbesondere eines Heimbeatmungsgerätes. Die Erfindung betrifft zudem ein Beatmungssystem zum Durchführen eines solchen Verfahrens.

Eine Heimbeatmung bietet mitunter den Vorteil, dass der Patient nicht in eine Klinik muss und zu Hause in seiner gewohnten Umgebung bleiben kann. Allerdings bedeutet dies auch, dass der Patient das Beatmungsgerät weitgehend selbstständig bedienen muss. Daher sind Beatmungsgeräte bekannt geworden, welche auch für Benutzer ohne medizinisches bzw. technisches Vorwissen ein hohes Maß an Bedienkomfort bieten und ohne ständige Überwachung durch medizinisches Personal zu Hause eingesetzt werden können.

Dabei hat sich jedoch gezeigt, dass es bei der Heimbeatmung neben der eigentlichen Bedienung des Beatmungsgerätes oft viele weitere Anlässe gibt, warum der Benutzende einen Kontakt zum Betreuer benötigt oder wünscht. Das betrifft mitunter die Kontrolle des Therapieverhaltens und beispielsweise wie lange und häufig das Beatmungsgerät eingesetzt wird. Ein weiterer Aspekt ist, dass die Motivation für die Therapie durch den seltenen Kontakt zum Betreuer schnell abnehmen kann. Dies ist jedoch sehr wichtig für den therapeutischen Erfolg. Ein weiteres Problem in der Heimbeatmung ist zudem, dass Sorgen und Nöte oder auch Wünsche erst dann wieder vorgetragen werden können, wenn ein Besuch beim Betreuer bzw. Arzt möglich ist. So gehört es beispielsweise zu den Sorgen und Nöten, dass das Einschlafen mit einer Atemmaske häufig sehr schwer fällt.

Demgegenüber ist es die Aufgabe der vorliegenden Erfindung, eine verbesserte Unterstützung eines Benutzenden eines Beatmungsgerätes und insbesondere eines Heimbeatmungsgerätes zur Verfügung zu stellen. Dabei soll die Unterstützung eine Selbsthilfe fördern bzw. eine Kontaktierung eines Betreuers wenigstens teilweise ersetzen oder diese vorbereiten.

Diese Aufgabe wird gelöst mit einem Verfahren nach Anspruch 1 sowie mit einem Beatmungssystem gemäß Anspruch 30. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Verfahren dient zur (insbesondere telematischen) Unterstützung eines Benutzenden eines Beatmungsgeräts und insbesondere eines Heimbeatmungsgerätes und/oder Schlaftherapiegeräts. Das Verfahren umfasst wenigstens die nachfolgenden Schritte (in dieser oder einer anderen sinnvollen Reihenfolge). Koppeln wenigstens eines mobilen Endgeräts mit dem Beatmungsgerät (mittels jeweils einer Übertragungseinrichtung) zum Austausch von Daten zwischen dem Beatmungsgerät und dem Endgerät. Das Endgerät umfasst insbesondere wenigstens einen Steueralgorithmus. Insbesondere ist der Steueralgorithmus auf dem Endgerät installiert und beispielsweise als eine Anwendung bzw. App (Application) ausgebildet. Übertragen von auf dem Beatmungsgerät registrierten Daten an das Endgerät. Die Daten umfassen wenigstens einen durch das Beatmungsgerät (insbesondere sensorisch mittels Sensormitteln) erfassten Beatmungsparameter. Auswerten der übermittelten Daten auf dem Endgerät und/oder auf dem Beatmungsgerät insbesondere mittels einer Prozessoreinrichtung des Endgeräts und/oder mittels des Steueralgorithmus. Zusätzlich oder alternativ kann das Auswerten der übermittelten Daten mittels wenigstens einer mit dem Endgerät oder dem Beatmungsgerät kommunizierenden Rechnereinrichtung und insbesondere eines Servers erfolgen. Ein solches Auswerten dient zur Bereitstellung wenigstens einer (insbesondere unabhängig von einem medizinischen Vorwissen verständlichen) Benutzerinformation. Das ist insbesondere derart, dass der Benutzende unabhängig von seinem medizinischen Fachwissen eine wenigstens teilweise auf dem Beatmungsparameter basierende und/oder eine wenigstens teilweise den Beatmungsparameter berücksichtigende und/oder eine wenigstens die Benutzung des Beatmungsgerätes und/oder die Therapie betreffende Bewertung und/oder Hilfestellung erhält. Ausgeben und vorzugsweise Anzeigen der Benutzerinformation mittels des Endgeräts wenigstens an den Benutzenden. Das Ausgeben erfolgt insbesondere mittels einer Ausgabeeinrichtung des Endgeräts. Insbesondere umfasst die Ausgabeeinrichtung wenigstens eine Anzeigeeinrichtung beispielsweise ein Display oder dergleichen.

In einer weiteren Ausgestaltung können die Daten zusätzlich von dem Endgerät an das Beatmungsgerät übertragen werden, wobei die Daten vom Endgerät und/oder von einem Hilfsgerät lokal erhoben werden.

Das Endgerät kann auch als Kommunikationshub beziehungsweise Modem für das Beatmungsgerät dienen. Das Endgerät kann Daten wie beispielsweise Atemflusskurven, Atemfrequenz, aktuelle Therapiedrücke, aktuelle Leckage und ähnliches vom Beatmungsgerät empfangen und weiterleiten.

Beispielsweise kann das Endgerät die Daten intern an eine andere Anwendung weiterleiten, zum Beispiel über eine SDK/API und/oder sonstige Software-Schnittstelle, die diese Daten auswertet. Alternativ kann das Endgerät die Daten über eine andere Schnittstelle des Endgerätes an ein Hilfsgerät weiterleiten. Hilfsgeräte können beispielsweise ein Diagnosegerät oder ein Datenrecorder in der Umgebung des Patienten sein. In manchen Ausführungsformen kann das Endgerät die Daten auch über eine Fernkommunikation (zum Beispiel Mobilfunk oder WLAN) an ein Hilfsgerät einer Gegenstelle weiterleiten, zum Beispiel an eine Cloud-Software oder eine Arzt-Software.

Das Endgerät kann umgekehrt auch unterschiedliche Daten empfangen und an das Beatmungsgerät weiterleiten.

Diese Daten können beispielsweise Daten sein, die lokal erhoben werden. Lokal erhobene Daten können beispielweise von mindestens einem Hilfsgerät erhoben werden. Hilfsgeräte können beispielsweise Diagnosegeräte sein. Hilfsgeräte können auch Thermometer oder Luftfeuchtemesser oder dergleichen sein, die Daten über Raumtemperatur und Luftfeuchte und dergleichen liefern.

Alternativ können es auch Daten sein, die das Endgerät aus der Ferne empfängt. Diese Daten können beispielsweise geänderte Beatmungsparameter sein, die der medizinische Betreuer hinterlegt und die das Endgerät von einem Speicherort und/oder vom Beatmungsgerät empfängt.

Die Daten können auch allgemeine Daten sein. Allgemeine Daten können beispielsweise den am Aufenthaltsort des Patienten zuständigen Betreuer umfassen, der dem Patienten angezeigt wird. Allgemeine Daten können auch Wetterdaten am Aufenthaltsort des Patienten umfassen, welche zu einer Optimierung der Beatmungs- und/oder Atemluftbefeuchtungs-und/oder Atemlufterwärmungs-Parameter genutzt werden können.

Mit diesen vom Endgerät gesammelten und an das Beatmungsgerät weitergeleiteten Daten kann das Beatmungsgerät seine Therapieparameter optimieren, nämlich zum Beispiel Therapiedrücke und/oder Triggerparameter und/oder Parameter des Atemluftbefeuchters beziehungsweise der Erwärmung der Atemluft.

Alternativ kann das Endgerät die optimierten Parameter anhand der Daten auch selbst ermitteln und dem Beatmungsgerät mitteilen.

Wenn Beatmungsgerät und Endgerät verbunden sind, beispielsweise per Bluetooth, kann das Beatmungsgerät und/oder das Endgerät parallel nach weiteren möglichen Kommunikationspartnern suchen. Beispielsweise kann das Endgerät einen zweiten Bluetooth-Kanal umfassen, mit dem es nach weiteren Kommunikationspartnern sucht.

Ein weiterer Kommunikationspartner kann beispielsweise ein Diagnosegerät sein wie z.B. Pulsoximeter, CO2-Messgeräte oder Polygraphen.

Ist ein weiterer Kommunikationspartner gefunden, kann sich dieses direkt mit dem Endgerät und/oder dem Beatmungsgerät verbinden. Bevorzugt findet die Verbindung direkt mit dem Beatmungsgerät statt.

Wenn ein weiterer Kommunikationspartner gefunden wird, kann das Endgerät dem Beatmungsgerät mitteilen, dass es diesen Kommunikationspartner gibt und eine Beendigung der Verbindung zwischen Endgerät und Beatmungsgerät veranlassen. So kann das Beatmungsgerät anschließend eine Verbindung zu dem weiteren Kommunikationspartner aufbauen.

Das erfindungsgemäße Verfahren bietet viele Vorteile. Einen erheblichen Vorteil bietet die Kommunikation zwischen Beatmungsgerät und Endgerät und die dadurch auf dem Endgerät erzeugte Benutzerinformation. Dadurch wird die Betreuung bei einer Heimtherapie erheblich verbessert und vereinfacht. Zum .Beispiel muss das Fachpersonal seltener besucht bzw. vor Ort sein. Besonders vorteilhaft ist dabei, dass die Unterstützung auch für medizinische bzw. technische Laien verständlich ist.

Mit der Erfindung kann sich der Patient in vielen Fällen besonders komfortabel selbst helfen. Zudem wird er besser in die Therapie eingebunden und hat die Möglichkeit, selbstständig die Therapie bzw. sein Verhalten bewerten und verbessern zu können. Besonders bei chronisch Kranken, welche die notwendige Überwachung durch Ärzte oft als unerwünschte Abhängigkeit empfinden, kann sich das positiv auf die Therapie auswirken und deren Selbstbewusstsein stärken.

Ein weiterer Vorteil der Erfindung ist, dass Endgeräte in der Regel leistungsstarke Prozessoren und hochauflösende Displays aufweisen. Daher eignen sich diese für die Auswertung und Erzeugung der Benutzerinformation besonders gut. Zugleich können Prozessorleistung und Display bei dem Beatmungsgerät schlichter gehalten werden. Dadurch kann wiederum die Laufzeit akkubetriebener Beatmungsgeräte erheblich verlängert werden. Zudem können Kosten eingespart und zugleich eine zuverlässige und sichere Beatmung gewährleistet werden. Mit dem Endgerät erhält der Benutzende daher einen besseren Einblick in seine Therapie und kann seine Sorgen und Nöte mitteilen sowie eine Bewertung des Therapieverhaltens bekommen. Das wäre sonst nur durch einen Besuch bzw. eine Kommunikation mit einem Betreuer möglich.

Es ist möglich und vorteilhaft, dass für die Bereitstellung der Benutzerinformation wenigstens eine hinterlegte therapeutische Zielsetzung berücksichtigt wird. Die Zielsetzung ist insbesondere wenigstens zeitweise auch in dem Endgerät hinterlegt. Insbesondere wird die Zielsetzung durch den Betreuer erstellt. Dadurch kann eine besonders gezielte Bewertung bzw. Hilfestellung erfolgen.

Die Bewertung bewertet vorzugsweise ein Therapieverhalten des Benutzenden. Es ist bevorzugt, dass dazu eine Häufigkeit und/oder Dauer und/oder Intensität der therapeutischen Nutzung des Beatmungsgeräts ausgewertet werden. Insbesondere werden sogenannte Compliance-Parameter (also insbesondere die Übereinstimmung des Patienten mit seiner Therapie und deren Anforderungen) ausgewertet. Dazu werden diese Parameter insbesondere automatisch durch das Beatmungsgerät erfasst und registriert.

In allen Ausgestaltungen ist es bevorzugt, dass die Bewertung wenigstens eine Aussage zur Masken-Dichtigkeit (Leckage) und/oder zur Häufigkeit von Rest-Atemereignisse (AHI) umfasst. Insbesondere wird dazu die Anzahl und/oder Häufigkeit der AHI angezeigt. Für die Masken-Dichtigkeit wird insbesondere eine die Masken-Dichtigkeit qualitativ charakterisierende Bewertung ausgegeben (z. B. von gut bis schlecht mit Abstufungen). Eine solche Bewertung kann auch grafisch erfolgen, z. B. anhand von Sternchen oder Smileys.

Es ist möglich und vorteilhaft, dass die Bewertung wenigstens ein Punktesystem und/oder Notensystem und/oder wenigstens ein Bewertungssystem mit (grafischen und/oder animierten) Symbolen und/oder wenigstens ein Belohnungssystem umfasst. Möglich sind auch Kombinationen solcher Systeme. So kann die Motivation für die Therapie weiter erhöht werden.

Vorteilhaft und bevorzugt ist, dass eine Belohnung des Belohnungssystems in Abhängigkeit von einem Erreichen einer hinterlegten therapeutischen Zielsetzung erfolgt. Insbesondere berücksichtigt das Belohnungssystem ein Maß für eine Abweichung des realen Therapieverhaltens des Benutzenden von der hinterlegten therapeutischen Zielsetzung. Es kann als Zielsetzung auch ein zuvor definiertes Therapieverhalten hinterlegt werden. Insbesondere ist die Belohnung symbolisch. Die Belohnung kann auch an andere Funktionen gekoppelt sein. Beispielsweise können als Belohnung Gaming-Funktionen freigeschaltet werden. Insbesondere werden mit dem Belohnungssystem Verbesserungen des Therapieverhaltens belohnt. Das bietet eine besonders anschauliche Rückmeldung über das Therapieverhalten, ohne dass erneut ein Besuch bei dem Betreuer erfolgen muss.

Vorzugsweise wird bei der Bewertung wenigstens eine vorhergehende Bewertung berücksichtigt. Möglich ist auch, dass wenigstens eine durch den Benutzenden erstellte bzw. hinterlegte (freiwillige) Zielsetzung berücksichtigt wird. So können je nach vorhergehender Bewertung bzw. freiwilliger Zielsetzung Fortschritte besonders gut erkannt werden.

Vorzugsweise umfasst die Hilfestellung wenigstens eine Anleitung zur Verwendung des Beatmungsgeräts und/oder des Endgeräts und/oder wenigstens eine (insbesondere für Laien optimierte) Therapierläuterung. Vorzugsweise wird die Hilfestellung durch wenigstens ein Video und/oder wenigstens eine Animation bereitgestellt oder umfasst wenigstens eine solche. Die Hilfestellung kann durch wenigstens ein gezieltes Verbinden mit einem Videoportal und/oder zu einem Video auf einem Server erfolgen.

In einer vorteilhaften Ausgestaltung ist vorgesehen, dass als Hilfestellung wenigstens ein Report für einen Betreuer erzeugt und insbesondere auf einen definierten Server geladen wird. Möglich ist auch, dass als Hilfestellung ein Hinzuziehen eines Betreuers empfohlen wird. So kann der Benutzende zügig und ohne einen zusätzlichen Besuch eine Rückmeldung bzw. weitere Hilfestellungen bekommen.

Der Report kann die Benutzerinformation und/oder weitere Daten umfassen. Die weiteren Daten dienen insbesondere dazu, die dem Benutzenden in der Benutzerinformation bereitgestellten Daten mit medizinischen und/oder technischen Informationen für den Betreuer zu ergänzen. Es ist möglich, dass der Report und/oder die weiteren Daten nur für den Betreuer einsehbar sind. Es ist möglich, dass der Betreuer automatisch eine Nachricht über den erstellten bzw. hinterlegten Report bekommt. Der Betreuer kann einen Zugang zu dem Server bekommen, auf welchem der Report hinterlegt ist. Beispielsweise ist ein solcher Server eine Cloud oder Teil einer solchen. Der Report ist insbesondere derart ausgebildet, dass der Betreuer über den vorliegenden Sachverhalt informiert wird. Zum Beispiel wird über die vom Beatmungsgerät, Endgerät und/oder Benutzenden erkannten Probleme oder auch Fortschritte informiert.

Das Hinzuziehen des Betreuers kann vorgeschlagen und/oder automatisch ausgeführt werden. Beispielsweise kann dazu ein Anruf erfolgen oder ein Termin mit dem Betreuer vereinbart werden. Dazu kann ein im Endgerät hinterlegter Kalender herangezogen werden. Möglich ist auch das Absetzen eines Notrufs. Insbesondere wird dabei die Schwere des der Hilfestellung zugrunde liegenden Problems berücksichtigt.

Es ist möglich und bevorzugt, dass der Betreuer über eine Datenübertragungsverbindung und beispielsweise ein Remoteverfahren auf das Endgerät und/oder das Beatmungsgerät zugreifen kann. Insbesondere kann der Betreuer dort weitere Daten abfragen und/oder Einstellungen vornehmen. Möglich ist auch, dass der Betreuer Nachrichten und/oder Terminvereinbarungen an das Endgerät senden kann. So kann über das Endgerät zunächst automatisch ermittelt werden, um welche Art von Anliegen oder Problemen es sich handelt. Sollte dann dennoch der Kontakt zu einem Betreuer notwendig sein, kann dieser dann weiterhelfen bzw. gezielt nachfragen.

In einer besonders bevorzugten und vorteilhaften Ausgestaltung ist auf dem Endgerät wenigstens eine Funktion für ein digitales Tagebuch hinterlegt. Insbesondere kann der Benutzende über das Tagebuch seine Erfahrungen mit der Therapie protokollieren. Insbesondere wird das Tagebuch für die Erstellung der Benutzerinformation und insbesondere der Hilfestellung und/oder für den Report und/oder für die Bewertung berücksichtigt.

In allen Ausgestaltungen ist es besonders bevorzugt, dass auf dem Endgerät wenigstens eine Eingabemaske angezeigt wird. In die Eingabemaske kann der Benutzende subjektive und/oder objektive Zusatzinformationen eingeben. Insbesondere erfolgt die Bereitstellung der Benutzerinformation wenigstens teilweise in Abhängigkeit der Zusatzinformation. Insbesondere werden für die Bereitstellung der Benutzerinformation die Zusatzinformationen mit den vom Beatmungsgerät bereitgestellten Daten und insbesondere dem Beatmungsparameter verrechnet. Zur Bereitstellung der Benutzerinformation können die Zusatzinformationen und die vom Beatmungsgerät bereitgestellten Daten gewichtet berücksichtigt werden. Die Benutzerinformation wird insbesondere in Abhängigkeit der Zusatzinformation und auch der vom Beatmungsgerät bereitgestellten Daten berechnet. Vorzugsweise wird die Benutzerinformation nach einer validierten Logik, welche insbesondere Teil des Steueralgorithmus ist, aus den Zusatzinformationen und den vom Beatmungsgerät bereitgestellten Daten berechnet.

Es ist bevorzugt und vorteilhaft, dass die Zusatzinformationen zeitabhängig und/oder aufgrund eines Steuerbefehls des Beatmungsgeräts und/oder aufgrund einer Anforderung durch den Benutzenden abgefragt werden. Eine zeitabhängige Abfrage erfolgt insbesondere in bestimmten Intervallen und/oder bei bestimmten Ereignissen und beispielsweise bei Einschalten des Beatmungsgerätes. Ein Steuerbefehl, welcher eine solche Abfrage auslöst, kann beispielsweise durch einen Bedienfehler und/oder eine Störung und/oder ein erkanntes besonderes Atemereignis erfolgen. Beispielsweise können bei Erkennen von Atemaussetzern, Undichtigkeiten an der Maske und/oder seltener Benutzung oder dergleichen Zusatzinformationen abgefragt werden. Eine Abfrage aufgrund einer Anforderung durch den Benutzenden kann beispielsweise erfolgen, wenn dieser einen einen Hilfe-Button drückt.

Die subjektiven Zusatzinformationen sind insbesondere solche, welche sensorisch nicht erfasst werden und/oder nicht erfassbar sind. Insbesondere umfassen die Zusatzinformationen ein Auftreten von Druckstellen und/oder Anpassungsschwierigkeiten bei Atemschnittstellen. Die subjektiven Zusatzinformationen sind insbesondere aus einer Gruppe von Zusatzinformationen entnommen, wenigstens umfassen: Unzufriedenheiten mit der Therapie, Schwierigkeiten mit dem Gerät, Handhabungs-Probleme, empfundene Nebenwirkungen, empfundene Druckstellen, Geräuschempfinden, Gesundheitsempfinden, Schlafgewohnheiten. Zusätzlich oder alternativ kann die Gruppe von subjektiven Zusatzinformationen wenigstens auch umfassen: Atemnot allgemein, Atemnot in spezifischen Situationen wie Liegen, Sitzen, Gehen, Essen, Treppelaufen, Laufen, am Morgen, am Abend, in der Nacht, nach Ausschalten des Beatmungsgerätes etc.; Sekretbildung in der Nase; Sekretbildung im Rachen; Sekretbildung in den oberen Atemwegen; Hustenkompetenz; Lebensqualität allgemein, krankheitsbezogene Lebensqualität, Müdigkeit am Tage, Schläfrigkeit am Tage. Dazu kann auch ein Wert auf einer Skala für Zufriedenheit angegeben werden.'

Die objektiven Zusatzinformationen sind insbesondere aus einer Gruppe von Zusatzinformationen entnommen, wenigstens umfassend: Ruhepuls, Belastungspuls, Alter, Gewicht, Körpergröße, Body Mass Index (BMI), Blutdruckwerte, Vorerkrankungen, Aktivität, Bewegung, Schlafverhalten (Schlafaktivität). Zusätzlich oder alternativ kann die Gruppe von objektiven Zusatzinformationen wenigstens auch umfassen: Lungenfunktion, insbesondere exspiratorischer Peakflow, Sauerstoffsättigung des Blutes, CO2-Gehalt des Blutes, CO2-Gehalt der Ausatemluft, organische Zusammensetzung der Ausatemluft (insbesondere volatile organische Moleküle), Schlagvolumen des Herzens, Minutenvolumen des Herzens, Herzfrequenz, Körpertemperatur, Umgebungsbedingungen (Temperatur, Luftdruck, Luftverschmutzung), Farbe des Sputum, Zusammensetzung des Sputum, Schlafqualität, Schlafstadien, Schlaffragementierung, Schlaf-/Wach-Information. Die objektiven Zusatzinformationen werden insbesondere sensorisch erfasst und/oder sind sensorisch erfassbar. Solche sensorisch erfassten Parameter können manuell eingegeben werden und/oder maschinell (in die App) eingelesen werden, z. B. durch eine Bluetooth-Verbindung, einer Kopplung der App auf dem Endgerät, z. B. über eine SDK, oder ein Einlesen der Sensordaten von einem Cloud-Server über API.

Es ist möglich, dass die objektiven Zusatzinformationen wenigstens teilweise durch das Endgerät und/oder das Beatmungsgerät bereitgestellt werden. Dazu sind insbesondere Sensormittel und/oder Gesundheits-Applikationen des Endgeräts abrufbar. Möglich und bevorzugt ist auch, dass die objektiven Zusatzinformationen vom Benutzenden selbst gemessene Gesundheitsparameter sind, beispielsweise Blutdruckwerte.

Die Eingabemaske umfasst vorzugsweise wenigstens einen Fragebogen mit einer Mehrzahl hinterlegter Fragen. Insbesondere werden die Fragen interaktiv und/oder dynamisch ausgewählt.

In beispielhaften Ausgestaltungen der Eingabemaske und insbesondere des Fragebogens werden die folgenden Schritte alle oder wenigstens teilweise durchgeführt:
- Bei einer zu geringen Nutzung (vorzugsweise unter 6 Stunden pro Tag, besonders bevorzugt unter 4 Stunden am Tag bzw. wahlweise auch unter 2 Stunden am Tag) oder einem Rückgang der Nutzung (vorzugsweise um mehr als 1 Stunde, besonders bevorzugt um mehr als 2 Stunden am Tag) wird gezielt nach einer fehlenden Motivation gefragt und/oder eine Aufklärung über den Sinn der Therapie angeboten und/oder nach möglichen Handhabungs-Problemen bei der Therapie und/oder nach möglichen Nebenwirkungen der Therapie und/oder nach einem möglichen Infekt der oberen Atemwege und/oder vermehrter Sekretbildung, die eine Atmung mit Atemmaske erschweren, gefragt.
- Bei einer steigenden oder erhöhten Maskenleckage (vorzugsweise Leckage-Median über 20 l/min oder über 15 l/min oder 95. Perzentil der Leckage über 50 l/min oder Anteil mit hoher Leckage höher als 10% der Therapiezeit) wird gezielt nach möglichen Leckage-bedingten Nebenwirkungen gefragt. Dabei wird besonders bevorzugt der Schwellwert der Leckage, der die gezielten Fragen auslöst, sensitiver gewählt, wenn eine niedrigere oder fallende Nutzungszeit pro Tag gemessen wird.
- Bei einer erhöhten Atemfrequenz (vorzugsweise über 20 bpm oder über 25 bpm) oder einer ansteigenden Atemfrequenz (vorzugsweise um über 3 bpm gegenüber einem Vergleichswert aus einem zeitlich früher liegenden Vergleichszeitraum) wird nach einer erhöhten oder gestiegenen Atemnot gefragt.

Vorzugsweise wird die Eingabemaske, insbesondere der Fragebogen, in Abhängigkeit wenigstens des vom Beatmungsgerät erfassten Beatmungsparameters und/oder einer zuvor bereitgestellten Benutzerinformation und/oder wenigstens einer Benutzereingabe erstellt und/oder angepasst. Die Eingabemaske kann auch aufgrund eines Steuerbefehls des Beatmungsgerätes und/oder anderer vom Beatmungsgerät bereitgestellten Daten erstellt bzw. angepasst werden. Eine solche Benutzereingabe kann beispielsweise eine Betätigung eines Hilfe-Buttons und/oder eine bestimmte Menüauswahl auf dem Endgerät sein. Solche Buttons sind insbesondere an einer Bedieneinrichtung des Beatmungsgerätes angeordnet. Die Eingabemaske kann aus hinterlegten einzelnen Elementen erzeugt und/oder aus einer Vielzahl von hinterlegten Eingabemasken ausgewählt werden.

Für die Bereitstellung der Benutzerinformation werden insbesondere (im Steueralgorithmus) definierte Beatmungsparameter herangezogen. Dabei ist möglich und bevorzugt, dass diejenigen definierten Beatmungsparameter, welche für die Auswertung nicht verfügbar sind und/oder nicht von dem Beatmungsgerät bereitgestellt wurden bzw. werden können, durch (subjektive und/oder objektive) Zusatzinformationen substituiert und/oder ergänzt werden. Insbesondere werden die dazu notwendigen Zusatzinformationen gezielt mit der Eingabemaske abgefragt. Dazu wird die Eingabemaske insbesondere entsprechend angepasst. Das ermöglicht eine erhebliche Verbesserung der Bewertung und Hilfestellung. Insbesondere ist im Steueralgorithmus hinterlegt, welche Zusatzinformationen zum Substituieren bzw. Ergänzen notwendig sind.

Vorzugsweise werden die für die Bereitstellung der Benutzerinformation verwendeten Beatmungsparameter wenigstens teilweise in Abhängigkeit der Zusatzinformationen ausgewählt.

Insbesondere erfolgt die Bereitstellung der Benutzerinformation interaktiv. Insbesondere wird dazu die Benutzerinformation grafisch dargestellt. Insbesondere ist dazu während der Darstellung der Benutzerinformation die Eingabe der subjektiven Zusatzinformationen und/oder weiterer Benutzereingaben möglich. Insbesondere wird die Benutzerinformation durch solche Eingaben erneut erstellt und/oder dynamisch angepasst.

In allen Ausgestaltungen ist es besonders bevorzugt, dass die Benutzerinformation zeitabhängig und/oder aufgrund eines Steuerbefehls des Beatmungsgerätes und/oder aufgrund einer Anforderung durch den Benutzenden erstellt wird. Ein solches Erstellen der Benutzerinformation ist insbesondere wenigstens teilweise so ausgebildet, wie es zuvor für das Abfragen der Zusatzinformationen beschrieben wurde.

Insbesondere wird für die Bereitstellung der Benutzerinformation ein Zeitraum von einer Mehrzahl von Therapiestunden und/oder einer Mehrzahl von Therapietagen herangezogen. Möglich sind auch längere oder kürzere Zeiträume.

In einer besonders bevorzugten und vorteilhaften Ausgestaltung ist es möglich, dass die Bereitstellung der Benutzerinformation während einer laufenden Therapie erfolgt. Das ermöglicht eine besonders schnelle und zügige Rückmeldung bzw. Hilfe. Ein weiterer Vorteil ist, dass es erheblich komfortabler ist, während der Therapie das mobile Endgerät zu benutzen, als beispielsweise ein Menü an dem Beatmungsgerät auszuwählen, welches gerade die Therapie ausführt.

Insbesondere werden während einer laufenden Therapie fortlaufend Daten von dem Beatmungsgerät an das Endgerät übertragen. Insbesondere wird die Benutzerinformation damit fortlaufend aktualisiert. Unter fortlaufend wird insbesondere sowohl eine stetige Wiederholung als auch eine Wiederholung zu bestimmten Zeitpunkten oder eine unregelmäßige Wiederholung verstanden.

In einer besonders bevorzugten und vorteilhaften Ausgestaltung wird die Benutzerinformation bei einem Atemereignis und vorzugsweise bei jedem neuen Atemereignis aktualisiert. Ein solches Atemereignis umfasst insbesondere eine Einatmung und/oder eine Ausatmung. Möglich sind auch andere Atemereignisse, beispielsweise besonders schnelle oder langsame oder flache Atmung oder Atemaussetzer oder dergleichen. So erhält der Benutzende ein zügiges und beispielsweise in Echtzeit oder nahezu in Echtzeit ausgeführtes Feedback über seine Atmung. Dadurch kann die Atemunterstützung, welche durch das Beatmungsgerät erfolgt, gezielt durch Mithelfen des Benutzenden ergänzt werden. Das ist beispielsweise dann besonders vorteilhaft, wenn als Therapieziel eine Atmung ohne Beatmungsgerät vorgesehen ist.

In einer besonders vorteilhaften und bevorzugten Weiterbildung ist vorgesehen, dass die Benutzerinformation wenigstens teilweise mithilfe wenigstens einer Projektionseinrichtung des Endgeräts in die Umgebung projiziert wird, insbesondere sodass die Benutzerinformation auch ohne direkte Betrachtung des Endgeräts wenigstens teilweise optisch wahrnehmbar ist. Insbesondere umfasst das Endgerät wenigstens eine Projektionseinrichtung. Insbesondere umfasst das Endgerät wenigstens eine Anzeigeeinrichtung und insbesondere ein Display. Insbesondere wird die Projektionseinrichtung durch die Anzeigeeinrichtung bereitgestellt oder umfasst wenigstens eine solche. Möglich ist auch eine Projektionseinrichtung, welche wenigstens teilweise separat zur Anzeigeeinrichtung ausgebildet ist. Insbesondere wird die Benutzerinformation derart projiziert, dass nicht auf das Endgerät und insbesondere nicht auf dessen Anzeigeeinrichtungen geschaut werden muss, um die Benutzerinformation wahrzunehmen.

Möglich ist aber auch, dass die Benutzerinformation wenigstens teilweise auf eine Anzeigeeinrichtung des Endgeräts dargestellt wird. Dann kann der Benutzende beispielsweise das Endgerät in den Händen halten und bequem im Bett liegen und die Benutzerinformation auf seinem Smartphone verfolgen, ohne den Blick auf das Beatmungsgerät richten zu müssen.

Möglich und bevorzugt ist auch, dass die Benutzerinformation wenigstens teilweise akustisch und/oder haptisch ausgegeben wird. Dazu erfolgt beispielsweise eine Wiedergabe von Tönen und/oder Geräuschen und/oder Melodien und/oder anderer Arten von akustisch wahrnehmbaren Sequenzen. Die haptische Wiedergabe kann beispielsweise durch ein Vibrieren oder dergleichen erfolgen. Auch das ermöglicht eine Wahrnehmung der Benutzerinformation ohne einen direkten Blick auf das Endgerät.

Vorzugsweise werden mit der Projektionseinrichtung unterschiedliche Leuchteffekte in die Umgebung projiziert. Möglich ist auch, dass mit der Projektionseinrichtung visuelle Strukturen auf wenigstens einer Projektionsfläche der Umgebung erzeugt werden. Die Projektionsfläche ist beispielsweise eine Wand und/oder eine Decke eines Raumes. Dazu können beispielsweise unterschiedliche Farben und/oder Muster jeweils für Einatmung und Ausatmung vorgesehen sein. Möglich ist auch, dass jeweils für Einatmung und Ausatmung unterschiedliche akustische und/oder haptische Signale eingesetzt werden. So kann dem Benutzenden ein direktes Feedback gegeben werden, wie lange er einatmen bzw. ausatmen sollte, um ein gutes Ergebnis mit der Therapie erzielen zu können. Möglich ist auch, dass darüber angezeigt wird, wann die Atmung zu flach und/oder zu langsam erfolgt.

In einer ebenfalls bevorzugten und vorteilhaften Weiterbildung ist vorgesehen, dass die Benutzerinformation wenigstens ein Maß dafür anzeigt, wie stark sich ein Ist-Wert eines vom Beatmungsgerät (aktuell) für die Beatmung des Benutzenden (sensorisch) erfassten Beatmungsparameters von einem (im Beatmungsgerät) hinterlegten Soll-Wert des Beatmungsparameters unterscheidet. Als Beatmungsparameter können beispielsweise die Atemfrequenz oder andere geeignete Beatmungsparameter vorgesehen sein. Das Maß kann beispielsweise durch entsprechende Farben oder Töne oder dergleichen dargestellt werden. Es kann beispielsweise grünes Licht projiziert werden, wenn die Atmung den Soll-Werten besonders gut entspricht und rotes Licht, wenn die Atmung zu stark vom Soll-Wert abweicht.

Möglich ist auch eine Benutzerinformation, welche als Animation ausgebildet ist bzw. bewegte grafische Elemente umfasst. Dann kann der Benutzende beispielsweise sein Smartphone beobachten und mithilfe der Animation verfolgen, ob seine Atmung der Vorgabe entspricht. In der Animation kann das Maß beispielsweise durch die Position oder Farbe oder Größe von Objekten veranschaulicht werden. Das ist beispielsweise auch bei Kindern als Patienten von großem Vorteil.

Es ist bevorzugt und besonders vorteilhaft, dass das Maß als wenigstens eine Animation ausgebildet ist, welche durch den Atemvorgang des Benutzenden interaktiv beeinflusst werden kann, sodass der Benutzende über die Veränderung der Animation eine direkte Rückkopplung darüber erfährt. Insbesondere erfährt der Benutzende eine direkte Rückkopplung darüber, wie nah seine Atmung an der für eine Therapie erforderlichen Atmung ist. Eine solche Animation kann optische und/oder akustische und/oder haptische Elemente umfassen. Die Animation kann auch in der Art eines Computerspiels bzw. eines Games vorgesehen sein. So kann der Benutzende spielerisch seine Atmung therapieren und unkompliziert erkennen, ob und in welche Richtung er seine Atmung anpassen sollte (Gamification). Beispielsweise muss mittels der Atmung versucht werden, ein virtuelles Objekt durch eine animierte Umgebung zu steuern. Die Bewegung ist dann zum Beispiel an das Ein- und Ausatmen gekoppelt.

Vorzugsweise ist das Verfahren als eine Entspannungs- bzw. Einschlafhilfe bei einer Anwendung des Beatmungsgerätes und/oder als eine Lernhilfe zur Anwendung des Beatmungsgerätes ausgebildet. Insbesondere wird dabei durch das Beatmungsgerät wenigstens ein hinterlegtes und/oder einstellbares Atemmuster vorgegeben. Insbesondere wird wenigstens ein für das Atemmuster notwendiger Soll-Beatmungsparameter eingestellt. Insbesondere wird das vorgegebene Atemmuster durch die Benutzerinformation mittels des Endgeräts ausgegeben. Insbesondere wird wenigstens ein Ist-Beatmungsparameter des Benutzenden sensorisch (durch das Beatmungsgerät) erfasst. Vorzugsweise wird dabei das Atemmuster in Abhängigkeit davon (fortlaufend) angepasst, wie stark sich der Ist-Beatmungsparameter vom Soll-Beatmungsparameter unterscheidet. Insbesondere wird dabei auch das vorgegebene Atemmuster teilweise an das reale Atemmuster des Patienten um ein definiertes Maß angepasst, um den Benutzenden die Synchronisation seiner Atmung mit dem vorgegebenen Atemmuster zu erleichtern. So kann der Benutzende durch Beobachtung der Benutzerinformation kontrollieren, ob seine Atmung dem Atemmuster entspricht, während das Atemmuster zugleich auch (leicht) an sein Atemmuster angepasst wird. Eine Angleichung des vorgegebenen Atemmusters an das reale Atemmuster des Benutzenden findet insbesondere wenigstens einmal pro Atemzug statt.

Die Anmelderin behält sich vor, ein solches Verfahren als Entspannungs- bzw. Einschlafhilfe bei einer Anwendung des Beatmungsgerätes und/oder als eine Lernhilfe zur Anwendung des Beatmungsgerätes separat zu beanspruchen. Auch dieses erfindungsgemäße Verfahren löst die zuvor gestellte Aufgabe besonders vorteilhaft. Vorzugsweise ist ein solches Verfahren wenigstens teilweise so ausgebildet, wie es zuvor für das andere erfindungsgemäße Verfahren beschrieben wurde. Möglich und bevorzugt ist, dass die Merkmale beider Verfahren in sinnvoller Weise miteinander kombiniert und/oder ausgetauscht werden können. Insbesondere dient das Verfahren nicht zur eigentlichen Therapie, sondern nur zur Vorbereitung oder Erleichterung einer vorgesehenen Atemtherapie. Vorzugsweise ist das Atemmuster bzw. die vorgegebene Atmung einstellbar und/oder aus einer Gruppe von Atemmustern auswählbar. So kann der Benutzende je nach Übung oder Einschlafgewohnheiten oder Therapieerfordernissen die Übung nach seinen Wünschen anpassen.

Besonders bevorzugt definiert das Atemmuster, wann und/oder wie lange eingeatmet werden soll. Zusätzlich oder alternativ kann das Atemmuster definieren, wann und/oder wie lange ausgeatmet werden soll. Insbesondere wird dazu für das Einatmen und/oder Ausatmen jeweils ein optisches und/oder akustisches und/oder haptisches Signal zeitgesteuert mit dem Endgerät als Benutzerinformation ausgegeben. Dazu ist die Benutzerinformation insbesondere wie zuvor beschrieben ausgebildet. Besonders bevorzugt wird dazu die Projektionseinrichtung und/oder die Anzeigeeinrichtung eingesetzt.

Beispielsweise kann das Atemmuster durch Farbwechsel und/oder Animationen dargestellt werden. Zusätzlich oder alternativ kann das Atemmuster durch Töne bzw. Geräusche und beispielsweise Meeresrauschen oder Vogelstimmen oder Melodien dargestellt werden. Besonders bevorzugt kann das Atemmuster interaktiv durch den Benutzenden beeinflusst werden, beispielsweise wie es zuvor für die interaktive Animation beschrieben wurde. So kann eine richtige Atmung bzw. ein schnelles Einschlafen spielerisch erlernt werden (Gamification). Beispielsweise kann auch hier ein virtuelles Objekt durch eine animierte Umgebung mittels der Atmung, welche dem Atemmuster entsprechen sollte, gesteuert werden.

Das erfindungsgemäße Beatmungssystem umfasst wenigstens ein Beatmungsgerät und wenigstens ein mobiles Endgerät. Das Beatmungsgerät ist dazu geeignet und ausgebildet, nach wenigstens einem der zuvor beschriebenen erfindungsgemäßen Verfahren betrieben zu werden. Insbesondere ist das Beatmungssystem dazu geeignet und ausgebildet, wenigstens eines der erfindungsgemäßen Verfahren auszuführen. Auch das erfindungsgemäße Beatmungssystem löst die zuvor gestellte Aufgabe besonders vorteilhaft.

Insbesondere dient das Verfahren zur Unterstützung bei einer mittels des Beatmungsgerätes durchgeführten bzw. durchführbaren Atemtherapie. Insbesondere erfolgt die Unterstützung vor und/oder nach und/oder während der Benutzung.

Die Daten und insbesondere der Beatmungsparameter können in Form von Rohdaten (direkt) übermittelt werden. Die Daten und insbesondere der Beatmungsparameter können vor dem Übermitteln eine Verarbeitung erfahren, beispielsweise eine Umwandlung und/oder Selektion oder dergleichen. Insbesondere umfasst die Kopplung ein Sicherheitssystem. Beispielsweise müssen das Endgerät und das Beatmungsgerät wenigstens ein Mal in physischer Nähe sein. Insbesondere erfolgt ein erneutes Koppeln wenigstens teilweise automatisch, z. B. bei Anmeldung im gleichen (WLAN-) Netzwerk. Das Übertragen von auf dem Beatmungsgerät registrierten Daten an das Endgerät erfolgt insbesondere über eine gesicherte Verbindung.

Die Daten und insbesondere der Beatmungsparameter werden insbesondere mittels wenigstens eines Sensormittels des Beatmungsgeräts erfasst. Der Beatmungsparameter ist insbesondere aus einer Gruppe von Beatmungsparametern entnommen, umfassend wenigstens: Druck; Flow (Flussrate bzw. Volumenstrom); Atemfrequenz; Atemzugvolumen; ermittelte Atemstörungen; Beatmungsparameter, welche Gerätefunktionen betreffen und z. B. eingestellte Therapiemodi wie CPAP, APAP; zeitliche Erfassung der Benutzung (wann, wie lange); Störungen; erkannte Bedienungsfehler.

Die Benutzerinformation kann wenigstens eine Handlungsanweisung umfassen oder als eine solche ausgebildet sein. Die Benutzerinformation kann auch eine Aktion umfassen oder als eine solche ausgebildet sein. Als Aktion kann beispielsweise ein automatisches Absetzen eines Notrufs und/oder eine Kontaktierung zum Betreuer erfolgen. Möglich sind auch andere geeignete Aktionen und beispielsweise ein Kommunizieren mit einer anderen Rechnereinrichtung und/oder mit dem Beatmungsgerät. Möglich sind auch andere optische und/oder akustische und/oder haptische Aktionen. In allen Ausgestaltungen ist es besonders bevorzugt, dass die Benutzerinformation wenigstens grafisch aufbereitet und z. B. animiert wird. Insbesondere enthält die Benutzerinformation grafische Bestandteile und/oder Textbestandteile.

Es ist möglich, dass die Bereitstellung der Benutzerinformation wenigstens teilweise abhängig von Geräteeinstellungen und/oder Therapieeinstellungen des Beatmungsgeräts erfolgt. Insbesondere kann die Therapie des Beatmungsgerätes unabhängig von der Unterstützung und insbesondere von der Bereitstellung der Benutzerinformation ausgeführt werden. Insbesondere erfolgt die Ausgabe der Benutzerinformation unabhängig von und/oder zusätzlich zu einer Ansteuerung einer Beatmungseinrichtung des Beatmungsgeräts. Unter einer Bereitstellung der Benutzerinformation wird insbesondere eine Erzeugung einer solchen verstanden.

Das mobile Endgerät ist insbesondere ein Smartphone oder ein Tablet oder eine andere Art von Handheld bzw. mobilen Smart Devices. Solche Endgeräte sind zum Beispiel Handhelds (wie Smartphones, faltbare Smartphones oder Tablets bzw. Touch Pads) oder andere Arten von Smart Devices (wie zum Beispiel eine Smart Watch) oder auch Notebooks, faltbare Notebooks oder dergleichen.

Im Rahmen der vorliegenden Erfindung wird unter dem Benutzenden insbesondere diejenige Person verstanden, welche die Therapie bekommt. Der Betreuer gehört insbesondere zum medizinischen und/oder technischen Fachpersonal oder ist ein Arzt. Als Betreuer kann beispielsweise ein technischer Support und/oder ein medizinischer Support vorgesehen sein. Unter einer (Heim-) Beatmung werden insbesondere (alle) Therapieformen mit einer Überdrucktherapie verstanden. Insbesondere werden darunter wenigstens verstanden: Schlaftherapie (CPAP, Bilevel, ASV), Highflow-Therapie, Sauerstoff-Therapie, nichtinvasive Beatmung, invasive Beatmung, Mundstückbeatmung.

In allen Ausgestaltungen ist bevorzugt, dass das Beatmungsgerät mittels des Endgeräts wenigstens teilweise fernsteuerbar ist. Insbesondere ist vorgesehen, dass das Beatmungsgerät zur Durchführung einer Therapie mittels des Endgeräts gestartet und/oder gestoppt und/oder pausiert und/oder eingestellt werden kann.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Beatmungssystems in einer perspektivischen Ansicht;
- Fig. 2: ein rein schematisches Blockschaltbild der Erfindung; und
- Fig. 3-6: Screenshots eines Endgeräts.

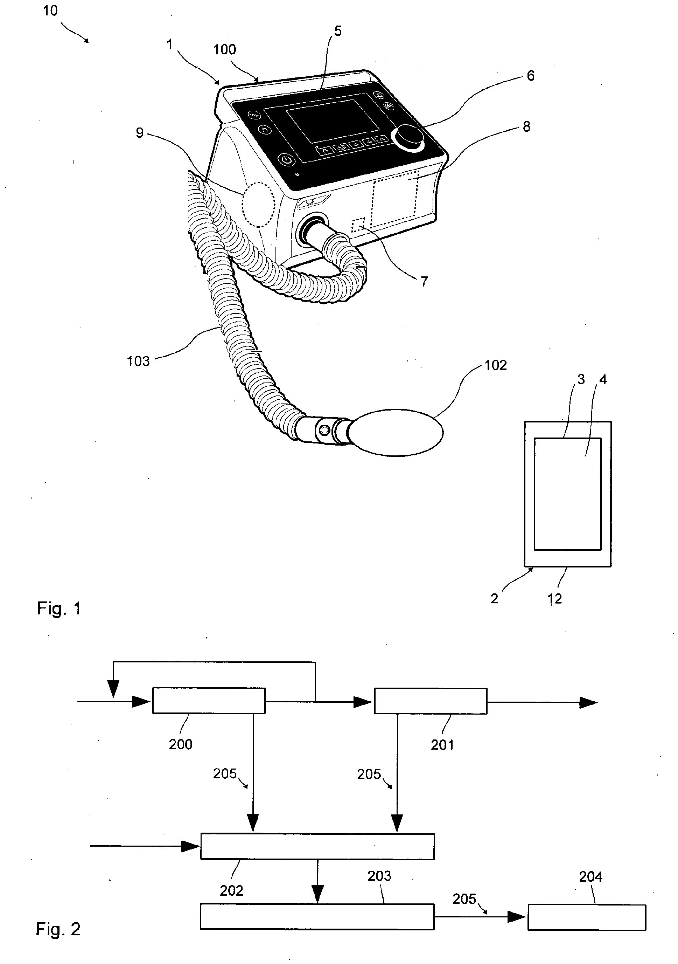

Die Figur 1 zeigt ein erfindungsgemäßes Beatmungssystem 10 mit einem als Heimbeatmungsgerät 100 ausgebildeten Beatmungsgerät 1 und einem Endgerät 2. Das Endgerät 2 ist hier als Smartphone 12 ausgebildet und umfasst eine als Anzeigeeinrichtung 4 mit einem Display. Das Endgerät 2 kann mit dem Beatmungsgerät 1 gekoppelt werden, beispielsweise über WLAN, Bluetooth oder eine andere Art kabelloser oder kabelgebundener Übertragungstechnik. Zwischen den gekoppelten Geräten 1, 2 des Beatmungssystems 10 können Daten übertragen werden. Das hier gezeigte Beatmungssystem 10 wird nach dem erfindungsgemäßen Verfahren betrieben.

Das Beatmungsgerät 1 weist im Inneren seines Gehäuses eine Beatmungseinrichtung 8 auf, welche mit einer Lüftereinrichtung 9 zur Erzeugung einer Atemgasströmung ausgestattet ist. Die Atemgasströmung wird über eine an die Beatmungseinrichtung 8 gekoppelte Schlaucheinrichtung 103 mit einer Atemschnittstelle 102 (z. B. Atemmaske) dem Benutzenden zugeführt.

Das Beatmungsgerät 1 umfasst hier eine Anzeigeeinrichtung 5 und eine Bedieneinrichtung 6. Dabei können auch Kombinationen von Bedieneinrichtung 6 und Anzeigeeinrichtung 5 vorgesehen sein, beispielsweise in der Art einer berührungsempfindlichen Anzeigefläche bzw. eines Touchscreens. Die Bedieneinrichtung 6 umfasst hier einen Hilfe-Button, auf den bei Problemen gedrückt werden kann, um Hilfe anzufordern. Zudem kann das Beatmungsgerät 1 hier teilweise von dem Endgerät 2 aus fernbedient werden.

Die Beatmungseinrichtung 8 ist hier mit einem Sensormittel 7 wirkverbunden, welches einen oder mehrere Sensoren zur Erfassung von Beatmungsparametern und ggf. weiterer für die Beatmung charakteristischer Größen aufweist. Zum Beispiel umfasst das Sensormittel 7 einen hier nicht näher gezeigten Drucksensor, welcher die Druckverhältnisse der Atemgasströmung erfasst. Das Sensormittel 7 ist hier mit einer Steuereinrichtung wirkverbunden, sodass die erfassten Beatmungsparameter registriert, ggf. ausgewertet und an das Endgerät 2 gesendet werden können. Als solche Beatmungsparameter können hier auch Geräteparameter und z. B. ein Akkustand oder eine Fehlermeldung oder dergleichen gesendet werden.

Über die Beatmungseinrichtung 8 können hier z. B. eine CPAP-Beatmung oder eine APAP-Beatmung ausgeführt werden. Für eine Beatmung wird die Beatmungseinrichtung 8 z. B. auf einen definierten Atemgasfluss und/oder ein Atemgasdruck eingestellt. Die Beatmungseinrichtung 8 kann einen notwendigen Minimaldruck bereitstellen und/oder Druckschwankungen kompensieren, die durch die Atemtätigkeit des Benutzers bedingt sind. Beispielsweise erfasst die Beatmungseinrichtung 8 mittels des Sensormittels 4 den gegenwärtigen Druck in der Atemmaske 102 und regelt die Leistung der Lüftereinrichtung 9 entsprechend nach, bis ein gewünschter Beatmungsdruck anliegt.

Das mit dem Beatmungsgerät 1 gekoppelte Endgerät 2 dient hier als eine Kommunikationsschnittstelle zwischen dem Benutzenden und dem.Beatmungsgerät 1 sowie zwischen dem Benutzenden und einem Betreuer bzw. Arzt. So kann der Benutzende telematisch unterstützt werden. Dazu ist ein Steueralgorithmus, hier eine Anwendung bzw. App, auf dem Endgerät installiert. Die auf dem Beatmungsgerät 1 registrierten Daten werden an das Endgerät 2 übertragen. Dann werden die übermittelten Daten auf dem Endgerät 2 ausgewertet, um eine Benutzerinformation bereitzustellen. Durch die Benutzerinformation erhält der Benutzende unabhängig von seinem medizinischen Fachwissen eine die übertragenden Beatmungsparameter berücksichtigende Bewertung und Hilfestellung. Die Benutzerinformation wird dann auf der Anzeigeeinrichtung 4 des Endgeräts 2 angezeigt.

Durch das Endgerät 2 mit der App können die Daten des Gerätes 1 interpretiert werden und der Benutzende bzw. Patient erhält nach einer validierten Logik Feedback zu seiner Therapie. Smartphones haben meist starke Prozessoren und gute Displays für eine solche Auswertung und sind für eine interaktive Präsentation der Auswertung besonders geeignet. Die Prozessorleitung und die Anzeige beim Beatmungsgerät 1 können dann eher schlicht gehalten werden, was Kosten spart und trotzdem eine gute und sichere Beatmung gewährleisten hilft.

In bestimmten Fällen (bestimmte Zeitpunkte, bei Verdacht auf Problemen, wenn der Patient auf den "Problem-Button" klickt), kann hier zusätzlich ein Fragebogen für den Patienten in der App angeboten werden, welchen er ausfüllen soll. Die Angaben des Patienten, z. B. zu Handhabungs-Problemen oder Nebenwirkungen, kombiniert die App mit den Geräte-Daten, um ein noch gezielteres Feedback zu geben. Dies kann auch die Empfehlung beinhalten, sich beim Betreuer bzw. betreuenden Arzt zu melden. In diesem Fall kann die App auch noch einen Report erzeugen über die erfassten Daten, den der Patient dem Arzt zur Verfügung stellen kann.

Der Patient kann hier als Zusatzinformationen Gesundheitsdaten bzw. "Health"-Daten vom Endgerät 2 in die App laden oder eingeben. Beispielsweise Ruhe- und Belastungspuls, Alter, Gewicht, Vorerkrankungen, Aktivität, Bewegung, Schlafmerkmale etc. Diese Zusatzinformationen werden optional zusätzlich mit den Daten des Beatmungsgerätes 1 verrechnet, um die Qualität der Interpretation zu verbessern. Zudem können auch subjektive Zusatzinformationen eingegeben und berücksichtigt werden, wie z. B. die Zufriedenheit.

Mit dem Beatmungssystem 10 kann hier auch ein Verfahren umgesetzt werden, welches als eine Entspannungs- bzw. Einschlafhilfe bei einer Anwendung des Beatmungsgerätes 2 und/oder als eine Lernhilfe zur Anwendung des Beatmungsgerätes 2 ausgebildet ist. Dadurch erhält der Benutzende eine Entspannungs-/Einschlaf-Hilfe über Biofeedback in der App. Als Benutzerinformation wird hier ein Atemmuster visualisiert.

Das Endgerät 2 gibt dem Benutzenden dazu über die App z. B. ein entspannendes Atemmuster vor, z. B. Atemfrequenz 10 bpm mit 1/3 Einatemzeit und 2/3 Ausatemzeit. Dieses Muster wird jedoch nicht starr abgespielt, da das reale Atemmuster und das vorgegebene Atemmuster sonst mehr und mehr auseinander laufen würden. Stattdessen wird die Vorgabe mindestens einmal pro Atemzug mit dem realen Atemmuster synchronisiert, sodass der Patient mit seiner aktuellen Atmung "abgeholt" wird und jeweils ein Stück Richtung idealer Atmung "geschubst" wird.

Die Vorgabe des realen Atemmusters erfolgt hier mittels einer Projektionseinrichtung 3, welche z. B. durch die Anzeigeeinrichtung 4 bereitgestellt wird. Beispielsweise wird dazu die Helligkeit des Displays erhöht. Durch eine Einfärbung des Displays in unterschiedlichen Farben für Ein- und Ausatmung wird das Atemmuster projiziert. So entsteht bei liegendem Endgerät 2 an der Zimmerdecke oder Zimmerwand ein Muster, welches das reale Atemmuster vorgibt. So kann die Übung beispielsweise im Liegen erfolgen, ohne dass der Blick auf das Endgerät 2 gerichtet werden muss. Alternativ oder zusätzlich kann die Vorgabe des realen Atemmusters auch durch eine grafische Animation am Display des Endgeräts 2 und/oder durch eine akustische Animation erfolgen.

Die Figur 2 zeigt ein beispielhaftes und hier stark vereinfachtes Blockschaltbild einiger Elemente der App. Als Benutzerinformation werden hier täglich ein Tages-Feedback 200 und wöchentlich (z. B. Sonntags) ein Wochen-Report 201 erstellt und ausgegeben. Dabei können z. B. persönliche Wochenziele berücksichtigt und ggf. belohnt werden. Im Rahmen des Tages-Feedbacks 200 kann z. B. auch ein Therapie-Tagebuch mit täglicher Möglichkeit zur Vergabe eines Emojis samt eines Kurztextes bereitgestellt werden.

Als mögliche Bewertungsregel für den Tages-Feedback 200 kann z B. jeder Therapietag nach bestimmten Kriterien einzeln in vier oder mehr Kategorien eingeteilt werden, beispielsweise von exzellent bis schlecht. Anhand der Kategorie kann dann die Feedback-Meldung für den einzelnen Tag festgelegt werden. Die Einzeltage und ihre Kategorien werden dann rechnerisch analysiert, um Daten für den Wochen-Report 201 zu liefern.

Rückmeldungen kann es z. b. zu Compliance (Geräte-Nutzung), Masken-Dichtigkeit (Leckage) und Anzahl Rest-Atemereignisse (AHI)geben. Im Tages-Feedback 200 und/oder im Wochen-Report 201 können auch vom Benutzer vorgenommene Rückmeldungen berücksichtigt werden, z. B. dessen Sternchen-Bewertungen.

In bestimmten Intervallen und zum Beispiel alle sieben, 28 oder 84 Tage wird hier ein Therapie-Check 202 gestartet, welcher dazu eine Eingabemaske mit einem Fragebogen umfasst. Der Therapie-Check 202 kann auch aufgrund eines Triggers 205 (Auslösers) gestartet werden, z. B. wenn ein Hilfe-Button gedrückt wird. Als Trigger 205 können auch das Auftreten bestimmter (sensorisch erfasster) Parameter oder bestimmter kritischer subjektiver oder objektiver Zusatzinformationen herangezogen werden. Auch bei erkannten Problemen können ein solcher Therapiecheck-Fragebogen und/oder eine Kontaktierung des Arztes empfohlen werden. Dazu sind insbesondere Schwellwerte hinterlegt, sodass deren Überschreiten eine solche Empfehlung auslösen kann.

Der Therapie-Check 202 kann automatisch oder aufgrund einer Anforderung analysiert werden, um eine Auswertung 203 des Therapie-Checks 202 zu erhalten. Die Auswertung 203 kann Belohnungen, Empfehlungen (z. B. das Hinzuziehen des Betreuers) und/oder Hilfestellungen (Troubleshooting) umfassen.

Die Auswertung 203 des Therapie-Checks 202 wird dann alleine oder zusammen mit zum Beispiel einem Statusbericht und/oder Gerätedaten an den Betreuer bzw. Arzt gesendet. Zudem kann das Senden der Auswertung 203 auch durch einen geeigneten Trigger 205 ausgelöst werden.

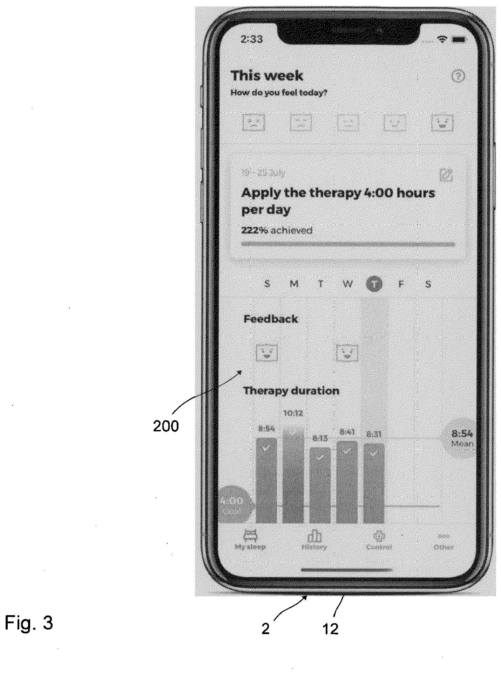

Die Figuren 3 bis 6 zeigen Screenshots (Bildschirmabbildungen) eines als Smartphone 12 ausgebildeten und mit der App ausgestatteten Endgeräts 2.

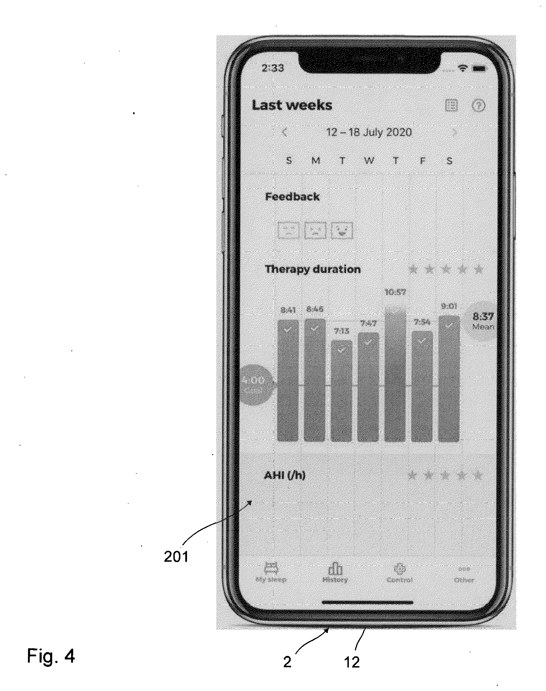

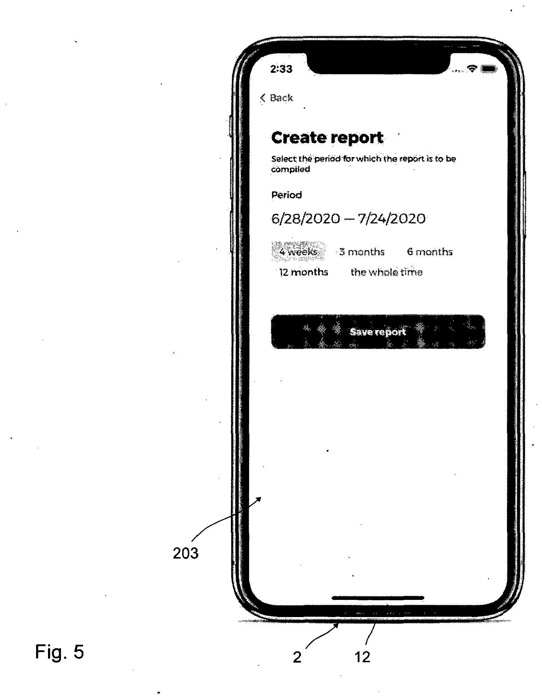

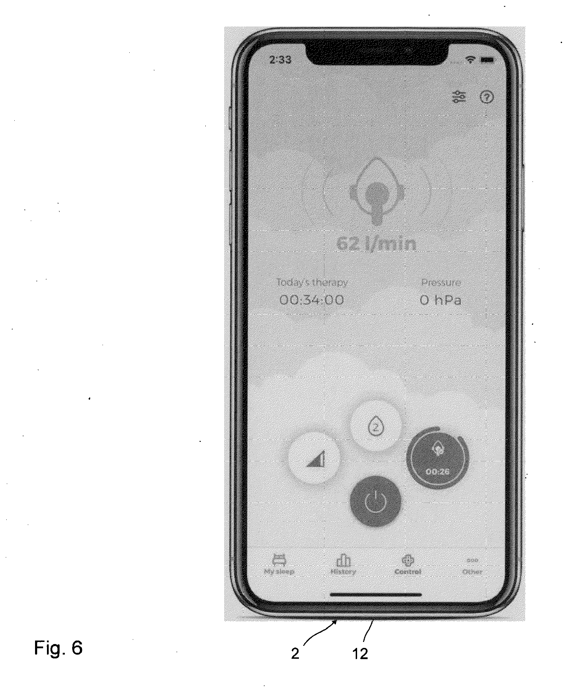

Die Figur 3 zeigt die Bereitstellung von Benutzerinformationen innerhalb einer Therapiewoche mit z. B. Tages-Feedbacks 200. In der Figur 4 ist ein Wochen-Report 201 gezeigt. Die Figur 5 zeigt den Bildschirm für eine Auswahl zum Starten einer Auswertung 203. In der Figur 6 ist der Bildschirm gezeigt, wenn das Endgerät 2 als Fernbedienung für das Beatmungsgerät 1 eingesetzt wird.

### Bezugszeichenliste:

- 1: Beatmungsgerät
- 2: Endgerät
- 3: Projektionseinrichtung
- 4: Anzeigeeinrichtung
- 5: Anzeigeeinrichtung
- 6: Bedieneinrichtung
- 7: Sensormittel
- 8: Beatmungseinrichtung
- 9: Lüftereinrichtung
- 10: Beatmungssystem
- 12: Smartphone
- 20: Hilfsgerät
- 100: Heimbeatmungsgerät
- 102: Atemschnittstelle
- 103: Schlaucheinrichtung
- 200: Tages-Feedback
- 201: Wochen-Report
- 202: Therapie-Check
- 203: Auswertung
- 204: Senden
- 205: Trigger

## Patentansprüche

1. Verfahren zur insbesondere telematischen Unterstützung eines Benutzenden eines Beatmungsgeräts (1), insbesondere eines Heimbeatmungsgeräts (100), umfassend wenigstens die folgenden Schritte:
- Koppeln wenigstens eines wenigstens einen Steueralgorithmus umfassenden mobilen Endgeräts (2) mit dem Beatmungsgerät (1) zum Austausch von Daten zwischen dem Beatmungsgerät (1) und dem Endgerät (2);
- Übertragen von auf dem Beatmungsgerät (1) registrierten Daten an das Endgerät (2), wobei die Daten wenigstens einen durch das Beatmungsgerät (1) erfassten Beatmungsparameter umfassen;
- Auswerten der übermittelten Daten auf dem Endgerät (2) und/oder auf dem Beatmungsgerät (1) und/oder mittels einer mit dem Endgerät (2) oder dem Beatmungsgerät (1) kommunizierenden Rechnereinrichtung zur Bereitstellung wenigstens einer Benutzerinformation, sodass der Benutzende unabhängig von seinem medizinischen Fachwissen eine wenigstens teilweise auf dem Beatmungsparameter basierende Bewertung und/oder Hilfestellung erhält;
- Ausgeben und insbesondere Anzeigen der Benutzerinformation mittels des Endgeräts (2).

2. Verfahren nach dem vorhergehenden Anspruch, wobei Daten zusätzlich von dem Endgerät (2) an das Beatmungsgerät (1) übertragen werden, wobei die Daten vom Endgerät (2) und/oder von einem Hilfsgerät (20) lokal erhoben werden.

3. Verfahren nach einem der vorhergehenden Ansprüche, wobei für die Bereitstellung der Benutzerinformation wenigstens eine hinterlegte therapeutische Zielsetzung berücksichtigt wird.

4. Verfahren nach einem der vorhergehenden Ansprüche, wobei die Bewertung ein Therapieverhalten bewertet und wobei dazu insbesondere eine Häufigkeit und/oder Dauer und/oder Intensität der therapeutischen Nutzung des Beatmungsgeräts (1) ausgewertet werden und/oder wobei die Bewertung wenigstens eine Aussage zur Masken-Dichtigkeit (Leckage) und/oder zur Häufigkeit von Rest-Atemereignisse (AHI) umfasst.

5. Verfahren nach einem der vorhergehenden Ansprüche, wobei die Bewertung ein Punktesystem und/oder Notensystem und/oder ein System mit grafischen Symbolen und/oder ein Belohnungssystem umfasst und wobei bei der Bewertung wenigstens eine vorhergehende Bewertung und/oder wenigstens eine durch den Benutzenden erstellte Zielsetzung berücksichtigt wird.

6. Verfahren nach einem der vorhergehenden Ansprüche, wobei die Hilfestellung wenigstens eine Anleitung zur Verwendung des Beatmungsgeräts (1) und/oder wenigstens eine Therapierläuterung umfasst und wobei die Hilfestellung insbesondere durch wenigstens ein Video und/oder eine Animation bereitgestellt wird und als Hilfestellung wenigstens ein Report für einen Betreuer erzeugt und insbesondere auf einen definierten Server geladen wird und/oder ein Hinzuziehen eines Betreuers empfohlen wird.

7. Verfahren nach einem der vorhergehenden Ansprüche, wobei auf dem Endgerät (2) wenigstens eine Funktion für ein digitales Tagebuch hinterlegt ist, über welches der Benutzende seine Erfahrungen mit der Therapie protokollieren kann und wobei das Tagebuch für die Erstellung der Hilfestellung und insbesondere für den Report und/oder für die Bewertung berücksichtigt wird.

8. Verfahren nach einem der vorhergehenden Ansprüche, wobei auf dem Endgerät (2) wenigstens eine Eingabemaske angezeigt wird, in welche der Benutzende subjektive und/oder objektive Zusatzinformationen eingeben kann und wobei die Bereitstellung der Benutzerinformation wenigstens teilweise in Abhängigkeit der Zusatzinformationen erfolgt.

9. Verfahren nach dem vorhergehenden Anspruch, wobei die subjektiven Zusatzinformationen sensorisch nicht erfasst werden und/oder nicht erfassbar sind und vorzugsweise ein Auftreten von Druckstellen und/oder Leckagen bei Atemschnittstellen (102) umfassen und/oder wobei die objektiven Zusatzinformationen aus einer Gruppe von Zusatzinformationen entnommen sind, wenigstens umfassend: Ruhepuls, Belastungspuls, Alter, Gewicht, Körpergröße, Body Mass Index (BMI), Blutdruckwerte, Vorerkrankungen, Aktivität, Bewegung, Schlafmerkmale.

10. Verfahren nach einem der beiden vorhergehenden Ansprüche, wobei die Eingabemaske wenigstens einen Fragebogen mit einer Mehrzahl hinterlegter Fragen umfasst und wobei die Fragen insbesondere interaktiv und/oder dynamisch ausgewählt werden.

11. Verfahren nach einem der drei vorhergehenden Ansprüche, wobei für die Bereitstellung der Benutzerinformation definierte Beatmungsparameter herangezogen werden und wobei diejenigen definierten Beatmungsparameter, welche für die Auswertung nicht verfügbar sind, durch Zusatzinformationen substituiert und/oder ergänzt werden und wobei die dazu notwendigen Zusatzinformationen gezielt mit der Eingabemaske abgefragt werden.

12. Verfahren nach einem der vier vorhergehenden Ansprüche, wobei die Bereitstellung der Benutzerinformation interaktiv erfolgt und wobei dazu die Benutzerinformation grafisch dargestellt wird und wobei während der Darstellung der Benutzerinformation die Eingabe der subjektiven Zusatzinformationen und/oder weiterer Benutzereingaben möglich ist und wobei die Benutzerinformation durch solche Eingaben dynamisch angepasst wird.

13. Verfahren nach einem der vorhergehenden Ansprüche, wobei die Benutzerinformation zeitabhängig und/oder aufgrund eines Steuerbefehls des Beatmungsgeräts (1) und/oder aufgrund einer Anforderung durch den Benutzenden erstellt wird, wobei für die Bereitstellung der Benutzerinformation ein Zeitraum von einer Mehrzahl von Therapiestunden und/oder von einer Mehrzahl von Therapietagen herangezogen wird.

14. Verfahren nach einem der vorhergehenden Ansprüche, wobei die Bereitstellung der Benutzerinformation während einer laufenden Therapie erfolgt, wobei während der laufenden Therapie fortlaufend Daten von dem Beatmungsgerät (1) an das Endgerät (2) übertragen werden und die Benutzerinformation damit fortlaufend aktualisiert wird, wobei die Benutzerinformation bei einem Atemereignis, umfassend insbesondere Einatmung und Ausatmung, aktualisiert wird.

15. Verfahren nach einem der vorhergehenden Ansprüche, wobei die Benutzerinformation mit Hilfe wenigstens einer Projektionseinrichtung (3) des Endgeräts (2) in die Umgebung projiziert wird, sodass die Benutzerinformation auch ohne direkte Betrachtung des Endgeräts (2) optisch wahrnehmbar ist und/oder wobei die Benutzerinformation akustisch und/oder haptisch erfolgt.

16. Verfahren nach einem der vorhergehenden Ansprüche, wobei die Benutzerinformation ein Maß dafür anzeigt, wie stark sich ein Ist-Wert eines vom Beatmungsgerät (1) aktuell für die Atmung des Benutzenden sensorisch erfassten Beatmungsparameters von einem für eine Therapie hinterlegten Soll-Wert des Beatmungsparameters unterscheidet.

17. Verfahren nach dem vorhergehenden Anspruch, wobei.das Maß als eine Animation ausgebildet ist und wobei die Animation durch den Atemvorgang des Benutzenden interaktiv beeinflusst werden kann, sodass der Benutzende über die Veränderung der Animation eine direkte Rückkopplung darüber erfährt, wie nah seine Atmung an der für eine Therapie erforderliche Atmung ist.

18. Verfahren nach einem der vorhergehenden Ansprüche, wobei das Verfahren als eine Entspannungs- bzw. Einschlafhilfe bei einer Anwendung des Beatmungsgeräts (1) und/oder als eine Lernhilfe zur Anwendung des Beatmungsgeräts (1) ausgebildet ist und wobei durch das Beatmungsgerät (1) wenigstens ein hinterlegtes und/oder einstellbares Atemmuster vorgegeben wird und wobei wenigstens ein für das Atemmuster notwendiger Soll-Beatmungsparameter eingestellt wird und wobei das vorgegebene Atemmuster insbesondere durch die Benutzerinformation mittels des Endgeräts (2) ausgegeben wird und wobei wenigstens ein Ist-Beatmungsparameter des Benutzenden sensorisch erfasst wird und wobei das Atemmuster in Abhängigkeit davon angepasst wird, wie stark sich der Ist-Beatmungsparameter vom Soll-Beatmungsparameter unterscheidet, sodass das vorgegebene Atemmuster teilweise an das reale Atemmuster des Patienten angepasst werden kann, um den Benutzenden die Synchronisation seiner Atmung mit dem vorgegebenen Atemmuster zu erleichtern.

19. Verfahren nach dem vorhergehenden Anspruch, wobei das Atemmuster definiert, wann und/oder wie lange eingeatmet und wann und/oder wie lange ausgeatmet werden soll und wobei dazu für das Einatmen und Ausatmen jeweils ein optisches und/oder akustisches und/oder haptisches Signal zeitgesteuert über das Endgerät (2) ausgegeben wird.

20. Beatmungssystem (10) mit wenigstens einem Beatmungsgerät (1) und mit wenigstens einem mobilen Endgerät (2), dazu geeignet und ausgebildet, nach dem Verfahren nach einem der vorhergehenden Ansprüche betrieben zu werden.
